# EUROPEAN PATENT SPECIFICATION

(11) **EP 0 346 612 B1**
(45) Date of publication and mention of the grant of the patent: **25.08.1993**
(21) Application number: 89108494.9
(22) Date of filing: 11.05.1989
(51) Int. Cl.: C07C 19/08, C07C 17/10

(54) **Process for production of 1,1,1-trifluoro-2,2-dichloroethane**
Verfahren zur Herstellung von 1,1,1-Trifluor-2,2-dichlorethan
Procédé de production de 1,1,1-trifluoro-2,2-dichloroéthane

(30) Priority: 17.05.1988 JP 120067/88
(43) Date of publication of application: 20.12.1989
(60) Divisional application: 92116814.2
(73) Proprietor: DAIKIN INDUSTRIES, LIMITED, Osaka-shi, Osaka-fu (JP)
(72) Inventor: Furutaka, Yasuhisa, Takatsuki-shi Osaka-fu (JP); Homoto, Yukio, Katano-shi Osaka-fu (JP); Honda, Tsunetoshi, Ageo-shi Saitama-ken (JP)
(74) Representative: Hansen, Bernd, Dr. Dipl.-Chem.

(56) References cited:
- US-A- 4 060 469
- US-A- 4 145 368

## Description

The present invention relates to a process for production of 1,1,1-trifluoro-2,2-dichloroethane. Particularly, the present invention relates to a process for 1,1,1-trifluoro-2,2-dichloroethane comprising chlorination of 1,1,1-trifluoro-2-chloroethane with chlorine gas in the presence or absence of a metal salt as a catalyst.

### Description of the Related Art

1,1,1-Trifluoro-2,2-dichloroethane is expected to replace trichlorofluoromethane, since it does not decompose ozone in the stratosphere. Then, it is desired to develop an economical process for the production of 1,1,1-trifluoro-2,2-dichloroethane.

Several processes for the production of 1,1,1-trifluoro-2,2-dichloroethane have been already proposed. For example, Czechoslovakian Patent No. 136,735 and Japanese Patent Kokai Publication No. 222038/1983 describe a process comprising reduction of 1,1,1-trifluoro-2,2,2-trichloroethane. Japanese Patent Publication No. 27375/1986 describes a process comprising isomerization of 1,1,2-trifluoro-1,2-dichloroethane. U.S. Patent No. 3,755,477 describes a process comprising fluorination of ethylene tetrachloride. Japanese Patent Kokai Publication No. 82711/1978 describes a process comprising photo chlorination of 1,1,1-trifluoro-2-chloroethane. McBee describes a process comprising chlorination of 1,1,1-trifluoroethane in J. Ind. Eng. Chem., 39, 409, (1947).

US-A-4 145 368 discloses a process for producing 1,1,1-trifluoro-2,2-dichloroethane which comprises chlorinating 1,1,1-trifluoro-2-chloroethane with chlorine in the absence of a catalyst.

However, from the points of view of the economical production, the above known processes for the production of 1,1,1-trifluoro-2,2-dichloroethane are not necessarily suitable since yield and selectivity through such the process are not high enough.

McBee describes that 1,1,1-trifluoroethane, which is a similar compound to a starting material for the production of 1,1,1-trifluoro-2,2-dichloroethane, reacts at the reaction temperature of 485 °C with chlorine at the molar ratio of 1 : 1. Under these conditions, the conversion of 1,1,1-trifluoroethane is 50 %. The product through this process contains 22.5 % of 1,1,1-trifluoro-2,2-dichloroethane and 41.8 % of 1,1,1-trifluoro-2,2,2-trichloroethane. It is reported that judging from the fact that the amounts of the compounds of which all hydrogen atoms are replaced with chlorine atoms have been increased, the chlorination rate increases as the chlorination proceeds, that is, as the number of hydrogen atoms replaced with chlorine atoms increases.

Accordingly, the above report indicates that the molar ratio of 1,1,1-trifluoro-2,2,2-trichloroethane to 1,1,1-trifluoro-2,2-dichloroethane is more than 1, that is, 1,1,1-trifluoro-2,2,2-trichloroethane is always produced more than 1,1,1-trifluoro-2,2-dichloroethane.

### SUMMARY OF THE INVENTION

It is, therefore, an object of the present invention to provide a commercially advantageous process for the production of 1,1,1-trifluoro-2,2-dichloroethane with overcoming the problems described above.

According to the present invention, there is provided a process for the production of 1,1,1-trifluoro-2,2-dichloroethane comprising the step of chlorinating 1,1,1-trifluoro-2-chloroethane with chlorine gas, characterized in that a metal salt is used as a catalyst for the reaction.

With the process of the present invention, the production ratio of 1,1,1-trifluoro-2,2-dichloroethane to 1,1,1-trifluoro-2,2,2-trichloroethane is about 1 : 0.3 at about 50 % conversion of 1,1,1-trifluoro-2-chloroethane and such the high production ratio cannot be expected from the report of McBee. Then, it becomes possible to produce 1,1,1-trifluoro-2,2-dichloroethane economically.

### DETAILED DESCRIPTION OF THE INVENTION

1,1,1-Trifluoro-2-chloroethane, as a starting material in the present process, can be easily produced by fluorination of trichloroethylene with anhydrous hydrogen fluoride in a liquid or gas phase.

The chlorination of the present invention proceeds with high selectivity when the metal salt is used as a catalyst.

The metal salt used in the present process is preferably metal fluoride such as CrF₃ or a metal chloride such as CuCl₂, NiCl₂ and FeCl₂.

It is preferable to use the metal salt carried on a support, for example, made of a metal oxide such as aluminum oxide or activated carbon. The most preferable catalyst includes NiCl₂, CuCl₂ and FeCl₂ carried on the support made of aluminum fluoride. Further, only the support can be used as a catalyst, that is, without the catalytic compound describe above, which also provides good production.

Generally, any type of the support can be used, and it is most preferable to use a spherical support or a pellet form support of which size is in the range of from 1 to 8 mm, particularly from 2 to 4 mm.

The amount of the catalyst carried on the support is suitably selected depending on the reaction conditions, the desired conversion and so on. Generally, the molar ratio of the metal salt as the catalyst to the metal oxide as the support is in the range of from 0.005 to 2, preferably from 0.01 to 1.0, for example, 0.03.

The reaction temperature of the chlorination of the present process is generally in the range of from 250 to 500 °C, preferably from 350 to 450 °C, for example 400 °C. The reaction is usually performed under the atmospheric pressure, although the present chlorination can be performed under pressure.

The molar ratio of chlorine gas to 1,1,1-trifluoro-2-chloroethane is preferably controlled in the range of from 0.05 to 0.5, particularly from 0.1 to 0.4 by taking account of the selectivity to 1,1,1-trifluoro-2,2-dichloroethane.

The contact time between the catalyst and the reactants can be suitably selected depending on the reaction conditions, especially the reaction temperature. Generally, the contact time is preferably controlled in the range of from 0.5 to 30 seconds, particularly from 1 to 25 seconds.

In the present process, any type of reactor, for example a tube reactor, can be used as long as the good contact between the catalyst and the reactants can be ensured.

In the process of the present invention, the reactor such as a tube reactor filled with the support carrying the catalyst is heated to a preselected temperature dependent on the reaction temperature, for example, in an electrical furnace. Then, 1,1,1-trifluoro-2-chloroethane and chlorine gas are supplied to the reactor to start the chlorination. The exit gas from the reactor is generally collected after water washing and drying steps.

In order to improve the conversion of 1,1,1-trifluoro-2-chloroethane, it is advantageous to recycle the unreacted 1,1,1-trifluoro-2-chloroethane to the reactor which is recovered from the top of a purification apparatus for purifying the produced gas.

Present invention will be hereinafter explained further in detail by following examples.

### Example 1

A tube reactor made of Hastelloy C (20 mm in inner diameter, 400 mm in length) was filled with 50 ml of spherical support particles (from 2 to 4 mm in diameter) made of AlF₃ carrying CuCl₂ in the molar ratio of 0.03 to AlF₃, and then heated to 370 °C in a nitrogen stream. After stopping the supply of nitrogen, 1,1,1-trifluoro-2-chloroethane and chlorine gas were supplied to the reactor at the flow rate of 100 ml/min. and 50 ml/min. (based on the standard conditions), respectively. The contact time based on the average residence time was about 8.5 seconds.

The exit gas from the tube reactor was collected after the water washing and the drying steps, and then analyzed with a gas chromatography. The conversion of 1,1,1-trifluoro-2-chloroethane was 45 % and the selectivity to 1,1,1-trifluoro-2,2-dichloroethane was 75 %.

### Comparative Examples 2-4

Using the same apparatus as Example 1, the process of the present invention was carried out without a catalyst. In these examples, 1,1,1-trifluoro-2-chloroethane was reacted with chlorine gas at the temperature of 400 °C with the ratio of the flow rate thereof as shown in Table 1. The exit gas from the reactor was analyzed as in Example 1.

The results are also shown in Table 1.

**Table 1**

| | Example 2 | Example 3 | Example 4 |
|---|---|---|---|
| Flow rate of 1,1,1-trifluoro-2-chloroethane | 90 ml/min. | 80 ml/min. | 70 ml/min. |
| Flow rate of chlorine gas | 10 ml/min. | 20 ml/min. | 30 ml/min. |
| Conversion of 1,1,1-trifluoro-2-chloroethane | 10 % | 22 % | 40 % |
| Selectivity to 1,1,1-trifluoro-2,2-dichloroethane | 91 % | 86 % | 77 % |

### Examples 5-9

Example 1 was repeated except that the metal salt shown in Table 2 carried on the AlF₃ support particles in the molar ratio of 0.03 was used and the reaction temperature was changed as shown in Table 2.

The results are also shown in Table 2.

**Table 2**

| | Example 5 | Example 6 | Example 7 | Example 8 | Example 9 |
|---|---|---|---|---|---|
| Catalyst | FeCl₂/AlF₃ | FeCl₂/AlF₃ | NiCl₂/AlF₃ | NiCl₂/AlF₃ | ---- |
| Reaction temperature | 330 °C | 370 °C | 330 °C | 370 °C | 450 °C |
| Conversion of 1,1,1-trifluoro-2-chloroethane | 32 % | 48 % | 26 % | 51 % | 79 % |
| Selectivity to 1,1,1-trifluoro-2,2-dichloroethane | 82 % | 72 % | 83 % | 71 % | 45 % |

### Comparative Example 1

1,1,1-Trifluoro-2-chloroethane and chlorine gas were supplied to a glass tube reactor (30 mm in inner diameter, 200 mm in length) made of Pyrex Glass® (commercially available from Corning Glass Works, U.S.) under the illumination of a high pressure mercury lamp of 100 wattages. Their flow rates were 100 ml/min. and 50 ml/min., respectively. The residence time in the reactor was about 60 seconds. The temperature in the reactor was 30 °C.

The produced gas discharged from the reactor was analyzed with the gas chromatography after water washing.

## Claims

1. A process for the production of 1,1,1-trifluoro-2,2-dichloroethane comprising the step of chlorinating 1,1,1-trifluoro-2-chloroethane with chlorine gas
characterized in that a metal salt is used as a catalyst for the reaction.

2. The process according to claim 1, in which said metal salt is selected from the group consisting of a metal fluoride and a metal chlorinate.

3. The process according to claim 1, in which said metal salt is carried on a support made of a material selected from the group consisting of a metal oxide, aluminum fluoride and activated carbon.

4. The process according to claim 1, in which aluminum fluoride is used as a catalyst.

## Patentansprüche

1. Verfahren für die Herstellung von 1,1,1-Trifluor-2,2-dichlorethan, umfassend die Stufe des Chlorierens von 1,1,1-Trifluor-2-chlorethan mit Chlorgas, dadurch **gekennzeichnet**, daß ein Metallsalz als Katalysator für die Reaktion verwendet wird.

2. Verfahren nach Anspruch 1, bei dem das Metallsalz aus der Gruppe aus einem Metallfluorid und einem Metallchlorinat ausgewählt wird.

3. Verfahren nach Anspruch 1, bei dem das Metallsalz auf einem Träger, der aus einem Material aus der Gruppe aus einem Metalloxid, Aluminiumfluorid und Aktivkohle hergestellt ist, getragen wird.

4. Verfahren nach Anspruch 1, bei dem Aluminiumfluorid als Katalysator verwendet wird.

## Revendications

1. Procédé pour la production de 1,1,1-trifluoro-2,2-dichloroéthane comprenant l'étape de chloration du 1,1,1-trifluoro-2-chloroéthane par le chlore gazeux, caractérisé en ce que l'on utilise un sel métallique comme catalyseur pour la réaction.

2. Procédé selon la revendication 1, dans lequel ledit sel métallique est choisi parmi les fluorures métalliques et les chlorures métalliques.

3. Procédé selon la revendication 1, dans lequel ledit sel métallique est porté sur un support choisi parmi les oxydes métalliques, le fluorure d'aluminium et le charbon actif.

4. Procédé selon la revendication 1, dans lequel on utilise le fluorure d'aluminium comme catalyseur.
